# EUROPEAN PATENT SPECIFICATION

(11) **EP 1 930 409 B1**
(45) Date of publication and mention of the grant of the patent: **08.02.2012**
(21) Application number: 06797011.1
(22) Date of filing: 23.08.2006
(51) Int. Cl.: C12N 1/21, C12N 15/09, C12P 13/14, C07K 14/34

(54) **L-GLUTAMIC ACID-PRODUCING BACTERIUM AND METHOD FOR PRODUCTION OF L-GLUTAMIC ACID**
L-GLUTAMINSÄURE PRODUZIERENDES BAKTERIUM UND VERFAHREN ZUR HERSTELLUNG VON L-GLUTAMINSÄURE
BACTERIE PRODUISANT DE L'ACIDE L-GLUTAMIQUE ET PROCEDE DE PRODUCTION DE L'ACIDE L-GLUTAMIQUE

(30) Priority: 26.08.2005 JP 2005245214
(43) Date of publication of application: 11.06.2008
(73) Proprietor: Ajinomoto Co., Inc., Tokyo 104-8315 (JP)
(72) Inventor: HIRANO, Seiko, Kawasaki-shi, Kanagawa 210-8681 (JP); NAKAMURA, Jun, Kawasaki-shi, Kanagawa 210-8681 (JP); ITO, Hisao, Kawasaki-shi, Kanagawa 210-8681 (JP)
(74) Representative: HOFFMANN EITLE
(86) International application number: PCT/JP2006/317036
(87) International publication number: WO 2007/024010

(56) References cited:
- EP-A- 0 469 517
- EP-A- 1 108 790
- WO-A1-95/23224
- DE-A1- 10 128 510
- JP-A- 2002 191 370
- US-A1- 2005 153 402
- KIMURA E: "METABOLIC ENGINEERING OF GLUTAMATE PRODUCTION" ADVANCES IN BIOCHEMICAL ENGINEERING, BIOTECHNOLOGY, SPRINGER, BERLIN, DE, vol. 79, 1 January 2003 (2003-01-01), pages 37-57, XP008048189 ISSN: 0724-6145
- DATABASE GENBANK [Online] 17 January 2003 NAKAGAWA S. ET AL.: 'Definition: Novel polynucleotide', XP002421299 Retrieved from NCBI Database accession no. (BD165509)
- KIMURA E. ET AL.: 'Coryneform Bacteria no Glutamic Acid Seisei Kiko-Maku Tokasei no Jubaku karano Kaiho-' PROTEIN, NUCLEIC ACID AND ENZYME vol. 42, no. 16, 1997, pages 2633 - 2640, XP003008696
- KIMURA E.: 'Triggering mechanism of L-glutamate overproduction by DtsR1 in coryneform bacteria' J. BIOSCI. BIOENG. vol. 94, no. 6, 2002, pages 545 - 551, XP002415658
- SHIRAI T. ET AL.: 'Comparative study of flux redistribution of metabolic pathway in glutamate production by two coryneform bacteria' METAB. ENG. vol. 7, no. 2, March 2005, pages 59 - 69, XP004801707
- SAKURAI M. ET AL.: 'Glutamic Acid o Kajo Seisei shiteiru Corynebacterium glutamicum no Morateki Idenshi Hatsugen Kaiseki' JAPAN SOCIETY FOR BIOSCIENCE, BIOTECHNOLOGY, AND AGROCHEMISTRY 2005 NENDO TAIKAI KEON YOSHISHU 05 March 2005, page 76 + ABSTR. NO. 29F2548, XP003008697

### SEQUENCE LISTING

<110> Ajinomoto Co., Inc.
<120> L-Glutamic acid producing bacterium and method for production of L-glutaroic acid
<130> 2005-151
<150> JP2005-245214 <151> 2005-08-26
<160> 6
<170> PatentIn version 3.1
<210> 1
   <211> 40
   <212> DNA
   <213> Artificial
<220>
   <223> primer for amplifying fasR
<400> 1
   gccgggtacc gctgccacaa atcgggcaag gataatctgc 40
<210> 2
   <211> 40
   <212> DNA
   <213> Artificial
<220>
   <223> primer for amplifying fasR
<400> 2
   gccgggtacc tcaaattccg gagttgagat ggagaaaact 40
<210> 3
   <211> 1020
   <212> DNA
   <213> Corynebacterium glutamicum
<220>
   <221> CDS
   <222> (506)..(976)
   <223> fasR
<400> 3
   gcctgccgtc cattctttac caactgttcg atttcttctt cacttcgagc agaacctgcc     60
   gcccgaccga tttcatcgcc ataaacccac aaaaccacac gtagatcctc gctgccacaa    120
   atcgggcaag gataatctgc gaggtgtccg tgatatttgc tcgcagtcac caagatgaac    180
   tcggcgtcac aaattgtgtc tttttgcacc aaccccgccc gaaactgacg gagagtctgc    240
   ctacgagcta gttggtgact gatctcattt tgatattgca ccacgattaa aaagtgtagc    300
   cgtaagccga ccaatgaaac gaccctctag aagcatcgtt tagaattgct tttaagtgaa    360
   taaggaacag cacagaatta aggcgtcgga gttatcgctc acgctacttc tcaagtggcg    420
   ccaaggtaag ttgtactttt tctgtccaaa ttattgtttt ttccgtagat aggttatcga    480
   acggaaatta cttggcaata ccgct atg ctg gca ggc atg cct aat tta aac       532
                               Met Leu Ala Gly Met Pro Asn Leu Asn
                               1               5
   gct gag gag cta gca gtc cgc gtg cga ccc gcg ctg aca aaa ctc tac       580
   Ala Glu Glu Leu Ala Val Arg Val Arg Pro Ala Leu Thr Lys Leu Tyr
   10                  15                  20                  25
   gtt ctc tat ttc cgc cgc tct gtg aat tct gac ctc tcg ggt cca cag       628
   Val Leu Tyr Phe Arg Arg Ser Val Asn Ser Asp Leu Ser Gly Pro Gln
                   30                  35                  40
   ctc act att ttg agt cgc ctg gaa gaa aac ggc cca tcc cga att agt       676
   Leu Thr Ile Leu Ser Arg Leu Glu Glu Asn Gly Pro Ser Arg Ile Ser
               45                  50                  55
   cgc atc gcg gaa ctt gaa gat att cgt atg cca acc gct tcg aat gct       724
   Arg Ile Ala Glu Leu Glu Asp Ile Arg Met Pro Thr Ala Ser Asn Ala
           60                  65                  70
   ctg cat cag ctg gag caa ctc aac ctg gtt gag cgt atc cgc gac acc       772
   Leu His Gln Leu Glu Gln Leu Asn Leu Val Glu Arg Ile Arg Asp Thr
       75                  80                  85
   aaa gac cgc cga ggc gtg cag gtt cag ctc act gat cat gga cgc gaa       820
   Lys Asp Arg Arg Gly Val Gln Val Gln Leu Thr Asp His Gly Arg Glu
   90                  95                  100                 105
   gag ctt gag cgc gtg aac aat gaa cga aac gca gag atg gct cga ctc       868
   Glu Leu Glu Arg Val Asn Asn Glu Arg Asn Ala Glu Met Ala Arg Leu
                   110                 115                 120
   ctt gaa atg ctc acc cca gag cag ctg gag cgt acc gaa gac ctg gtg       916
   Leu Glu Met Leu Thr Pro Glu Gln Leu Glu Arg Thr Glu Asp Leu Val
               125                 130                 135
   gat atc att act gag ctt gca gag gtg tac ggt agc tgg aaa gag acc       964
   Asp Ile Ile Thr Glu Leu Ala Glu Val Tyr Gly Ser Trp Lys Glu Thr
   140 145 150
   gac agc ggt tct taacagtttt ctccatctca actccggaat ttgatgaaac aacc 1020
   Asp Ser Gly Ser
   155
<210> 4
   <211> 157
   <212> PRT
   <213> Corynebacterium glutamicum
<400> 4
   Met Leu Ala Gly Met Pro Asn Leu Asn Ala Glu Glu Leu Ala Val Arg
   1 5 10 15
   Val Arg Pro Ala Leu Thr Lys Leu Tyr Val Leu Tyr Phe Arg Arg Ser
   20 25 30
   Val Asn Ser Asp Leu Ser Gly Pro Gln Leu Thr Ile Leu Ser Arg Leu
   35 40 45
   Glu Glu Asn Gly Pro Ser Arg Ile Ser Arg Ile Ala Glu Leu Glu Asp
   50 55 60
   Ile Arg Met Pro Thr Ala Ser Asn Ala Leu His Gln Leu Glu Gln Leu
   65 70 75 80
   Asn Leu Val Glu Arg Ile Arg Asp Thr Lys Asp Arg Arg Gly Val Gln
   85 90 95
   Val Gln Leu Thr Asp His Gly Arg Glu Glu Leu Glu Arg Val Asn Asn
   100 105 110
   Glu Arg Asn Ala Glu Met Ala Arg Leu Leu Glu Met Leu Thr Pro Glu
   115 120 125
   Gln Leu Glu Arg Thr Glu Asp Leu Val Asp Ile Ile Thr Glu Leu Ala
   130 135 140
   Glu Val Tyr Gly Ser Trp Lys Glu Thr Asp Ser Gly Ser
   145 150 155
<210> 5
   <211> 474
   <212> DNA
   <213> Corynebacterium glutamicum
<220>
   <221> CDS
   <222> (1).. (474)
   <223> fasR
<400> 5
   atg ctg gca ggc atg cct aat tta aac gct gag gag cta gca gtc cgc      48
   Met Leu Ala Gly Met Pro Asn Leu Asn Ala Glu Glu Leu Ala Val Arg
   1               5                   10                  15
   gtg cga ccc gcg ctg aca aaa ctc tac gtt ctc tat ttc cgc cgc tct      96
   Val Arg Pro Ala Leu Thr Lys Leu Tyr Val Leu Tyr Phe Arg Arg Ser
               20                  25                  30
   gtg aat tct gac ctc tcg ggt cca cag ctc act att ttg agt cgc ctg     144
   Val Asn Ser Asp Leu Ser Gly Pro Gln Leu Thr Ile Leu Ser Arg Leu
           35                  40                  45
   gaa gaa aac ggc cca tcc cga att agt cgc atc gcg gaa ctt gaa gat     192
   Glu Glu Asn Gly Pro Ser Arg Ile Ser Arg Ile Ala Glu Leu Glu Asp
       50                  55                  60
   att cgt atg cca acc gct tcg aat gct ctg cat cag ctg gag caa ctc     240
   Ile Arg Met Pro Thr Ala Ser Asn Ala Leu His Gln Leu Glu Gln Leu
   65                  70                  75                  80
   aac ctg gtt gag cgt atc cgc gac acc aaa gac cgc cga ggc gtg cag     288
   Asn Leu Val Glu Arg Ile Arg Asp Thr Lys Asp Arg Arg Gly Val Gln
                   85                  90                  95
   gtt cag ctc act gat cat gga cgc gaa gag ctt gag cgc gtg aac aat     336
   Val Gln Leu Thr Asp His Gly Arg Glu Glu Leu Glu Arg Val Asn Asn
               100                 105                 110
   gaa cga aac gca gag atg gct cga ctc ctt gaa atg ctc acc cca gag     384
   Glu Arg Asn Ala Glu Met Ala Arg Leu Leu Glu Met Leu Thr Pro Glu
           115                 120                 125
   cag ctg gaa cgt acc gaa gac ctg gtg gat atc att act gag ctt gca     432
   Gln Leu Glu Arg Thr Glu Asp Leu Val Asp Ile Ile Thr Glu Leu Ala
       130                 135                 140
   gag gtg tac ggt agc tgg aaa gag acc gac agc ggt tct taa             474
   Glu Val Tyr Gly Ser Trp Lys Glu Thr Asp Ser Gly Ser
   145                 150                 155
<210> 6
   <211> 157
   <212> PRT
   <213> Corynebacterium glutamicum
<400> 6
   Met Leu Ala Gly Met Pro Asn Leu Asn Ala Glu Glu Leu Ala Val Arg
   1               5                   10                  15
   Val Arg Pro Ala Leu Thr Lys Leu Tyr Val Leu Tyr Phe Arg Arg Ser
               20                  25                  30
   Val Asn Ser Asp Leu Ser Gly Pro Gln Leu Thr Ile Leu Ser Arg Leu
   35 40 45
   Glu Glu Asn Gly Pro Ser Arg Ile Ser Arg Ile Ala Glu Leu Glu Asp
   50 55 60
   Ile Arg Met Pro Thr Ala Ser Asn Ala Leu His Gln Leu Glu Gln Leu
   65 70 75 80
   Asn Leu Val Glu Arg Ile Arg Asp Thr Lys Asp Arg Arg Gly Val Gln
   85 90 95
   Val Gln Leu Thr Asp His Gly Arg Glu Glu Leu Glu Arg Val Asn Asn
   100 105 110
   Glu Arg Asn Ala Glu Met Ala Arg Leu Leu Glu Met Leu Thr Pro Glu
   115 120 125
   Gln Leu Glu Arg Thr Glu Asp Leu Val Asp Ile Ile Thr Glu Leu Ala
   130 135 140
   Glu Val Tyr Gly Ser Trp Lys Glu Thr Asp Ser Gly Ser
   145 150 155

## Description

### Technical Field

The present invention relates to fermentation industry, more precisely, it lelates to a method for producing L-glutamic acid and a coryneform bacterium used for it. L-Glutamic acid is widely used as a raw material of seasonings.

### Background Art

L-glutamic acid is conventionally produced by fermentation using coryneform bacteria belonging to the genus *Brevibacterium, Corynebacterium* or the like having an L-glutamic acid producing ability. In order to improve productivity of these coryneform bacteria, bacterial strains isolated from the nature, or artificial mutant strains or strains modified by gene recombination of them are used.

Wild type strains of coryneform bacteria do not produce L-glutamic acid under conditions that biotin is present in an excessive amount. Therefore, in the conventional L-glutamic acid production methods, either culturing in a medium in which biotin concentration is restricted, or, when a medium containing a sufficient amount of biotin is used, culturing in a medium to which a surfactant or lactam antibiotic is added as a biotin activity suppressing agent at the start of the culture or during the culture is employed. However, especially when a raw material which is inexpensive, but contains an excessive amount of biotin, such as blackstrap molasses, is used as a carbon source of the medium, the biotin activity suppressing agent, of which addition to the medium is required, invites high production cost (Non-patent document 1).

Meanwhile, existence of a gene (*dtsR* gene) encoding a protein (DTSR protein) which originates from a bacterium of the genus *Corynebacterium* and imparts resistance to a surfactant to the bacteria has been identified, and it has been elucidated that an L-glutamic acid producing coryneform bacterium in which that gene hhas been disrupted produces a marked amount of L-glutamic acid even under conditions that biotin is present in such an amount that wild strains scarcely produces L-glutamic acid, and that if the *dtsR* gene is amplified in a L-glutamic acid producing coryneform bacterium, the L-glutamic acid producing ability thereof is enhanced.

Moreover, the inventors of the present invention have found that if temperature sensitivity to biotin activity suppressing agent is imparted to an L-glutamic acid producing coryneform bacterium, the bacterium can stably produce L-glutamic acid by fermentation even in the presence of biotin, and that if L-lysine producing ability is imparted to such a strain showing temperature sensitivity to a biotin activity suppressing agent, the strain can stably produce L-lysine and L-glutamic acid simultaneously by fermentation even in the presence of biotin. They further disclose gene substitution using a variant *dtsR* gene encoding a temperature sensitive variant DTSR protein as one of means for imparting temperature sensitivity to biotin activity suppressing agent to coryneform bacteria (Patent document 2).

However, any gene which improves sensitivity to a surfactant via increase of expression amount thereof in coryneform bacteria has not been known to date.
Non-patent document 1: Biosci. Biotech. Biochem., 61 (7), 1109-1112, 1997
Non-patent document 2: Biochem. Biophys. Res. Commun., 1997 May 8, 234(1):157-61
Patent document 1: International Patent Publication WO95/23224
Patent document 2: International Patent Publication WO96/06180

### Disclosure of the Invention

An object of the present invention is to provide a novel technique for improving L-glutamic acid producing ability in the L-glutamic acid production using a coryneform bacterium.

The inventors of the present invention conducted various researches in order to achieve the aforementioned object. As a result, they found that by amplifying the *fasR* gene of coryneform bacteria in cells, L-glutamic acid producing ability thereof could be improved, and L-glutamic acid production was enabled in a biotin-rich medium without adding a biotin activity suppressing agent such as a surfactant, and thus accomplished the present invention.
1. A method for producing L-glutamic acid, comprising:
   (A) culturing a coryneform bacterium in a medium, and
   (B) collecting L-glutamic acid from the medium, wherein said coryneform bacterium is modified so that the expression of the fasR gene is enhanced by increasing the copy number of the gene or modifying an expression control sequence of the gene as compared to a parent strain, such that said bacterium is capable of producing L-glutamic acid in a medium containing biotin at a concentration of 50 µg/l or higher and not containing a biotin activity suppressing agent selected from the group consisting of surfactant and penicillin, and wherein the fasR gene is selected from the group consisting of:
      (a) a DNA comprising nucleotides 506 to 976 in SEQ ID NO: 3 or nucleotides 1 to 474 in SEQ ID NO: 5,
      (b) a DNA which is able to hybridize with nucleotides 506 to 976 in SEQ ID NO: 3 or nucleotides 1 to 474 in SEQ ID NO: 5 under stringent conditions comprising washing at 0.1 x SSC, 0.1% SDS at 60°C.
2. The method according to item 1, wherein said bacterium is Corynebacterium glutamicum.
3. The method according to any one of items 1 to 2, wherein said fasR gene has a homology of 95% or more to the entire amino acid sequence of SEQ ID NO: 4 ID.

### Brief Explanation of the Drawings

Fig. 1 shows the construction procedure of the plasmid pVKFASR for amplification *of fasR* gene.

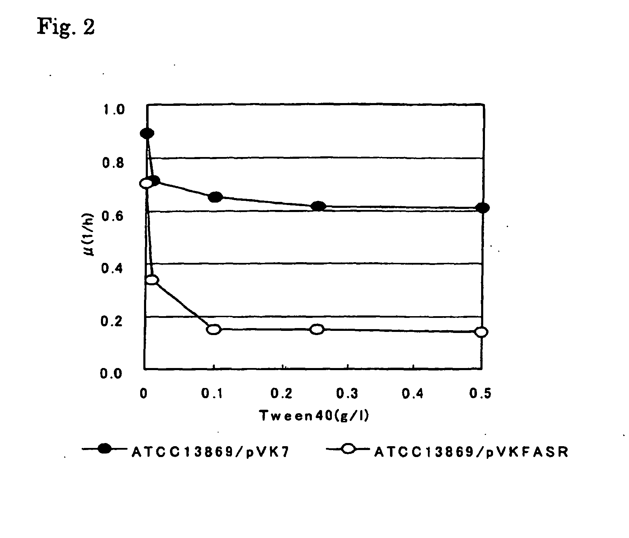

Fig. 2: A graph showing a growth curve of *a fasR* gene-amplified strain in the presence of a surfactant.

### Description of the Preferred Embodiments

### <1-1> Coryneform bacterium used for the present invention having L-glutamic acid producing ability

The coryneform bacterium used for the present invention is a coryneform bacterium which has L-glutamic acid producing ability, and which has been modified so that expression of the *fasR* gene is enhanced, and which shows improved sensitivity to a surfactant as compared with a strain of which *fasR* gene is not enhanced.

The coryneform bacteria used for the present invention include *Corynebacterium* bacteria and those bacteria having been previously classified into the genus *Brevibacterium* but have been re-classified into the genus *Corynebacterium* (Int. J. Syst. Bacteriol., 41, 255 (1981)), and further include bacteria belonging to the genus *Brevibacterium,* which is extremely close to the genus *Corynebacterium.* Specific examples include the followings.:
*Cornynebacterium acetoacidophilum*
*Corynebacterium acetoglutamicum*
*Corynebacterium alkanolyticum*
*Corynebacterium callunae*
*Corynebacterium glutamicum*
*Corynebacterium lilium*
*Corynebacterium melassecola*
*Corynebacterium thermoaminogenes (Corynebacterium efficiens)*
*Corynebacterium herculis*
*Brevibacterium divaricatum*
*Brevibacterium flavum*
*Brevibacterium immariophilum*
*Brevibacterium lactofermentum (Corynebacterium glutamicum)*
*Brevibacterium roseum*
*Brevibacterium saccharolyticum*
*Brevibacterium thiogenitalis*
*Brevibacterium ammoniagenes*
*Brevibacterium album*
*Brevibacterium cerinum*
*Microbacterium ammoniaphilum*

Specifically, the following strains can be encompassed:
*Corynebacterium acetoacidophilum* ATCC 13870
*Corynebacterium acetoglutamicum* ATCC 15806
*Corynebacterium alkanolyticum* ATCC 215 11
*Corynebacterium callunae* ATCC 15991
*Corynebacterium glutamicum* ATCC 13020, ATCC 13032, ATCC 13060
*Corynebacterium lilium* ATCC 15990
*Corynebacterium melassecola* ATCC 17965
*Corynebacterium thermoaminogenes* AJ12340 (FERM BP-1539)
*Corynebacterium herculis ATCC* 13868
*Brevibacterium divaricatum* ATCC 14020
*Brevibacterium flavum* ATCC 13826, ATCC 14067
*Brevibacterium immariophilum* ATCC 14068
*Brevibacterium lactofermentum* ATCC 13869 *(Corynebacterium glutamicum* ATCC 13869)
*Brevibacterium roseum* ATCC 13825
*Brevibacterium saccharolyticum* ATCC 14066
*Brevibacterium thiogenitalis* ATCC 19240
*Brevibacterium ammoniagenes* ATCC 6871, ATCC 6872
*Brevibacterium album* ATCC 15111
*Brevibacterium cerinum* ATCC 15112
*Microbacterium ammoniaphilum* ATCC 15354

These strains are available from the American Type Culture Collection. That is, each strain is given a registration number. Strains can be ordered using this registration number (http://www.atcc.org/). The registration number corresponding to each strain is listed in the catalogue of the ATCC. The AJ 12340 strain was deposited at National Institute of Bioscience and Human Technology, Agency of Industrial Science-and Technology, Ministry of International Trade and Industry (currently International Patent Organism Depositary, National Institute of Advanced Industrial Science and Technology at Tsukuba Central 6, 1-1, Higashi 1-chome, Tsukuba-shi, Ibaraki-ken 305-5466, Japan) on October 27, 1987 under the provisions of the Budapest Treaty and given an accession number of FERM BP-1539.

In the present invention, "L-glutamic acid producing ability" means an ability to accumulate L-glutamic acid in a medium or cells when the coryneform bacterium of the present invention is cultured in the medium. The bacterium of the present invention may have this L-glutamic acid producing ability as a property of a wild strain of the coryneform bacterium, or the ability may be a property imparted or enhanced by breeding. Furthermore, the L-glutamic acid producing ability may be imparted by enhancing expression of *the fair* gene in the manner described later.

Examples of the method for imparting or enhancing L-glutamic acid producing ability by breeding include enhancing expression of a gene encoding an enzyme involved in L-glutamic acid biosynthesis. Examples of the enzyme involved in L-glutamic acid biosynthesis include glutamate dehydrogenase, glutamine synthetase, glutamate synthase, isocitrate dehydrogenase, aconitate hydratase, citrate synthase, phosphoenolpyruvate carboxylase, pyruvate carboxylase, pyruvate dehydrogenase, pyruvate kinase, phosphoenolpyruvate synthase, enolase, phosphoglyceromutase, phosphoglycerate kinase, glyceraldehyde-3-phophate dehydrogenase, triose phosphate isomerase, fructose bisphosphate aldolase, phosphofructokinase, glucose phosphate isomerase, and so forth.

As for the methods for enhancing expression of these genes, the enhancement can be achieved by introducing an amplification plasmid obtained by introducing a DNA fragment containing any of these genes into a suitable plasmid, for example, a plasmid vector containing at least a gene responsible for replication and proliferation functions of the plasmid in coryneform bacteria, by increasing copy number of these genes in a chromosome by conjugation, gene transfer etc., or by introducing a mutation into a promoter region of these genes (refer to International Patent Publication WO95/34672). Examples of plasmids autonomously replicable in coryneform bacteria include, but not limited to, the plasmid pCRY30 described in Japanese Patent Laid-open No. 3-210184; the plasmids pCRY21, pCRY2KE, pCRY2KX, pCRY31, pCRY3KE and pCRY3KX described in Japanese Patent Laid-open No. 2-72876 and U.S. Patent No. 5,185,262; the plasmids pCRY2 and pCRY3 described in Japanese Patent Laid-open No. 1-191686; pAM330 described in Japanese Patent Laid-open No. 58-67679; pHM1519 described in Japanese Patent Laid-open No. 58-77895; pAJ655, pAJ611, and pAJ1844 described in Japanese Patent Laid-open No. 58-192900; pCG1 described in Japanese Patent Laid-open No. 57-134500; pCG2 described in Japanese Patent Laid-open No. 58-35197; pCG4 and pCG11 described in Japanese Patent Laid-open No. 57-183799, and so forth.

When an objective gene is introduced into the aforementioned amplification plasmid or chromosome, any promoter may be used as a promoter for expression of the gene so long as a promoter that functions in coryneform bacteria is chosen. The promoter may be the inherent promoter of the gene used, or a modified promoter. Expression of a gene can also be controlled by suitably choosing a promoter that potently functions in coryneform bacteria, or by approximating -35 and -10 regions of promoter to the consensus sequence. Examples of the coryneform bacteria modified so that expression of genes of citrate synthase, isocitrate dehydrogenase, pyruvate dehydrogenase, and/or glutamate dehydrogenase is enhanced include the microorganisms described in International Patent Publication WO00/18935, European Patent Publication No. 1010755, and so forth.

The modification for imparting the L-glutamic acid producing ability may also be attained by reducing or deleting activity of an enzyme that catalyzes a reaction branching from a biosynthetic pathway of L-glutamic acid to produce another compound. Examples of enzymes that catalyze a reaction branching from an L-glutamic acid biosynthesis pathway to synthesize a compound other than L-glutamic acid include isocitrate lyase, α-ketoglutarate dehydrogenase, acetyl phosphate transferase, acetate kinase, acetohydroxy acid synthase, acetolactate synthase, acetyl formate transferase, lactate dehydrogenase, glutamate decarboxylase, 1-pyrroline dehydrogenase, and so forth. Examples of coryneform bacteria of which α-ketoglutarate dehydrogenase activity is reduced include the following strains.
*Brevibacterium lactofermentum* ΔS strain (WO95/34672)
*Brevibacterium lactofermentum* AJ12821 strain (FERM BP-4172; see FR9401748)
*Brevibacterium flavum* AJ12822 strain (FERM BP-4173; see FR9401748)
*Corynebacterium glutamicum* AJ12823 strain (FERM BP-4174; see FR9401748)

In order to reduce or delete activity of any one of the enzymes mentioned above, a mutation that reduces or deletes the intracellular activity of the enzyme can be introduced into the gene of the aforementioned enzyme on a chromosome by a usual mutagenesis method. For example, it can be attained by deleting the gene encoding the enzyme on a chromosome, or modifying an expression control sequence such as promoter and Shine-Dalgarno (SD) sequence by gene recombination. Furthermore, it can also be attained by introducing a mutation for amino acid substitution (missense mutation), a stop codon (nonsense mutation), or a fi-ame shift mutation that adds or deletes one or two nucleotides into a region encoding the enzyme on a chromosome, or by deleting the gene partially or entirely (Journal of Biological Chemistry, 272:8611-8617 (1997)). Furthermore, the enzymatic activity can also be reduced or deleted by constructing a gene encoding a mutant enzyme of which coding region is deleted, and substituting the constructed gene for the normal gene on a chromosome by homologous recombination or the like, or by introducing a transposon or IS factor into the gene.

For introduction of such mutations to reducing or deleting activity of an enzyme as described above into a gene, for example, the following methods may be used. By modifying a partial sequence of an objective gene to prepare a mutant gene designed so that it does not produce an enzyme that functions normally, and transforming a coryneform bacterium with a DNA containing the gene to cause recombination of the mutant gene and the gene on a chromosome, the objective gene on a chromosome can be replaced with the mutant gene. Such site-specific mutagenesis based on gene substitution utilizing homologous recombination has already been established, and examples of the method therefor include the methods using a linear DNA or a plasmid containing a temperature sensitive replication origin (U.S. Patent No. 6,303,383, Japanese Patent Laid-open No. 05-007491), and so forth. Moreover, such site-specific mutagenesis based on gene substitution utilizing homologous recombination as described above can also be performed by using a plasmid not showing a replication ability in a host.

Examples of the temperature sensitive plasmid for coryneform bacterium include p48K and pSFKT2 (see U.S. Patent No. 6,303,383 for these), pHSC4 (see French Patent Laid-open No. 2667875, 1992 and Japanese Patent Laid-open No. 5-7491) and so forth These plasmids can autonomously replicate at 25°C at least, but cannot autonomously replicate at 37°C in coryneform bacteria. The *Escherichia coli* AJ12571 harboring pHSC4 was deposited at the National Institute of Bioscience and Human-Technology, the Agency of Industrial Science and Technology, the Ministry of International Trade and Industry (currently, the independent administrative corporation, National Institute of Advanced Industrial Science and Technology, International Patent Organism Depositary (Tsukuba Central 6, 1-1, Higashi 1-Chome, Tsukuba-shi, Ibaraki-ken, Japan, postal code: 305-5466)) on October 11, 1990, receiving an accession number of FERM P-11763. Then, the deposit was converted into an international deposit under the provisions of the Budapest Treaty on August 26, 1991, receiving an accession number of FERM BP-3524.

Other methods of imparting or enhancing the L-glutamic acid producing ability include imparting resistance to organic acid analogs or respiratory inhibitors, or sensitivity to inhibitor of cell wall synthesis. Examples of such methods include, for example, imparting resistance to benzopirone or naphtoquinones (JP56-1889A), imparting resistance to HOQNO (JP56-140895A), imparting resistance to α-ketomalonic acid (JP57-2689A), imparting resistance to guanidine (JP56-35981A), imparting sensitivity to penicillin (JP04-88994A), imparting daunomycin resistance (JP58-158192A), and so forth.

Specific examples of such resistant bacteria include the following strains:
*Brevibacterium flavum* AJ11355 (FERM P-5007; JP56-1889A)
*Corynebacterium glutamicum* AJ11368 (FERM P-5020; JP56-1889A)
*Brevibacterium flavum* AJ11217 (FERM P-4318; JP57-2689A)
*Corynebacterium glutamicum AJ11218* (FERM P-4319; JP57-2689A)
*Brevibacterium, flavum* AJ11564 (FERM P-5472; JP56-140895A)
*Brevibacteriumflavum* AJ11439 (FERM P-5136; JP56-35981A)
*Corynebacterium glutamicum* H7684 (FERM BP-3004; JP04-88994A)

A coryneform bacterium which is preferred to be used as a parent strain of the coryneform bacterium of the present invention is a strain which accumulates L-glutamic acid under conditions that biotin concentration in the medium is restricted, or conditions that the medium contains biotin at a high concentration and a surfactant or lactam antibiotic as a biotin activity suppressing agent. Here, examples of the surfactant include saturated fatty acids such as lauric acid, myristic acid, stearic acid and palmitic acid, fatty acid ester type nonionic surfactants such as glycerol fatty acid esters, sorbitan fatty acid esters, sucrose fatty acid esters, polyethylene glycol fatty acid esters, polyethylene glycol/polypropylene glycol fatty acid esters and polyoxyethylene sorbitan fatty acid esters, and N-acylamino acids such as N-palmitoylglycine, N-palmitoylalanine, N-palmitoylvaline, N-palmitoylleucine, N-palmitoylthreonine, N-palmitoylmethionine, N-palmitoylaspartic acid, N-palmitoylglutamic acid, N-myristoylglutamic acid, N-stearoylglutamic acid, N,N'-dipalmitoylomithine and N,N'-dipalmitoyllysine. Examples of the antibiotic include lactam antibiotics such as penicillin and cephaloridine (Yamada K., Takahashi J. & Nakamura J., Hakkokogaku Kaishi, 20, 348-350 (1962); Udagawa K., Abe S. & Kinoshita S., Hakkokogaku Kaishi, 40, 614-619 (1962)).

Here, the conditions that biotin concentration is restricted correspond to a biotin concentration in the medium of 30 µg/L or lower, preferably 20 µg/L or lower, more preferably 10 µg/L or lower, under an L-glutamic acid production condition, or the medium may not contain biotin at all. Under the conditions that the medium contains biotin at a high concentration and a surfactant or lactam type antibiotic as a biotin activity suppressing agent, the medium contains biotin at a concentration of 50 µg/L or higher, preferably 100 µg/L or higher, more preferably 200 µg/L or higher, concentration of the antibiotic as the biotin activity suppressing agent in the medium is 0.1 U/ml or higher, preferably 0.2 U/ml or higher, more preferably 0.4 U/ml or higher, and concentration of the surfactant as the biotin activity suppressing agent in the medium is 0.4 g/L or higher, preferably 1 g/L or higher, more preferably 2 g/L or higher. However, so long as L-glutamic acid production is induced, the concentrations of these substances are any concentration.

Examples of strains suitable as the strain which accumulates L-glutamic acid under the aforementioned L-glutamic acid production condition include, for example, wild type strains of the aforementioned coryneform bacteria and the following strains.
*Brevibacterium flavum* AJ11217 (FERM P-4318; JP57-2689A)
*Corynebacterium glutamicum* AJ11218 (FERM P-4319; JP57-2689A)
*Brevibacterium lactofermentum* AJ11426 (FERM P-5123 JP56-048890A)
*Corynebacterium glutamicum AJ11440* (FERM P-5137 JP56-048890A)
*Brevibacterium lactofermentum* AJ11796 (FERM P-6402 JP58-158192A)

### <1-2> Enhancement of fasR gene expression

The state of "modified so that expression of the *fasR* gene is enhanced" corresponds to a state that the number of the molecules of the FasR product per cell is increased as compared with that of the parent strain or a wild strain, a state that the activity of FasR product per molecule is increased, and so forth. The wild-type coryneform bacterium used as the reference for the comparison is, for example, *Corynebacterium glutamicum (Brevibacterium lactofermentum)* ATCC13869 or ATCC13032. If a coryneform bacterium is modified so that expression amount *of* the *fasR* gene increases, sensitivity to a surfactant of the coryneform bacterium increases.

In the present invention, the "activity which increases sensitivity to a surfactant" means the activity of the *fasR* gene product of the present invention. Specifically, it means an activity of enabling L-glutamic acid production in a medium containing biotin at a high concentration by increasing expression amount of the *fasR* gene. The "medium containing biotin at a high concentration" referred to here is a medium having a biotin concentration of 50 µg/L or higher, preferably 100 µg/L or higher, more preferably 200 µg/L or higher, and not containing a biotin activity suppressing agent such as penicillin or surfactant, or containing it at a reduced concentration. As for the biotin activity suppressing agent, a surfactant is desirably added at a concentration of 0.1 g/L or lower, preferably 0.05 g/L or lower, more preferably 0.01 g/L or lower, or it may not be added at all.

Furthermore, when the parent strain has an ability to accumulate L-glutamic acid in a medium containing biotin at a high concentration in the absence of a biotin activity suppressing agent, the L-glutamic acid productivity is improved by amplification of *the fasR* gene. Here, if a *fasR* gene-amplified strain shows an L-glutamic acid accumulation amount in a medium or L-glutamic acid production rate higher than those of a non-amplified strain, it can be said that the L-glutamic acid producing ability of the *fasR* gene-amplified strain is improved. It can be said that the L-glutamic acid producing ability is improved, if the yield per saccharide is improved by improvement of the expression of *the fasR* gene by, for example, 2% or more, desirably 4% or more, more desirably 6% or more, as compared with the parent strain or a non-modified strain.

Increase of the expression amount of *the fasR* gene can be confirmed by comparing amount of m-RNA *of fasR* with that of a wild-type or non-modified strain. Examples of the method for confirming the expression amount include Northern hybridization and RT-PCR (Molecular Cloning, Cold Spring Harbor Laboratory Press, Cold Spring Harbor (USA) 2001). Increase degree of the expression amount is not particularly limited so long as it increases as compared with that of a wild strain or non-modified strain. However, it is desirably increases, for example, 1.5 times or more, preferably 2 times or more, more preferably 3 times or more, as compared with a wild strain or non-modified strain.

The protein encoded by *the fasR* gene of the present invention is a kind of transcriptional regulator, or a homologue thereof, and is a protein showing an activity of improving sensitivity of coryneform bacteria to a surfactant when expression thereof is enhanced (Fatty Acid Synthase Regulator, fasR). Examples of the gene encoding fasR *(fasR* gene) of coryneform bacteria include, for example, DNA containing the nucleotide sequence of the nucleotide numbers 506 to 976 of SEQ ID NO: 3 derived from *C*. *glutamicum (B. lactofermentum*) ATCC 13869, and a gene having the nucleotide sequence of SEQ ID NO: 5 derived from *C*. *glutamicum* ATCC 13032. The *fasR* gene of *Corynebacterium glutamicum* ATCC 13032 is encoded by the nucleotides of 3189878 to 3190351 in the genome sequence registered as Genbank Accession No. NC_003450, and it is registered as NCgl 2886. Moreover, as the gene of the present invention, homologue genes of *fasR* derived from other microorganisms may also be used so long as sensitivity to a surfactant can be enhanced by amplification thereof in coryneform bacteria. Homologues of the *fasR* gene can be searched for with reference to the gene sequence of NCgl 2886 by using BLAST or the like (http://blast.genome.jpn.

Since the sequence of the *fasR* gene which can be used for the present invention has already been elucidated, *fasR* and a flanking region thereof including a control region of *fasR* can be obtained by PCR (polymerase chain reaction, see White, T.J. et al., Trends Genet., 5, 185 (1989)) using primers prepared on the basis of the elucidated nucleotide sequence, for example, the primers shown in SEQ ID NOS: 1 and 2 and chromosomal DNA of a coryneform bacterium as a template. Homologues *of fasR* of other microorganisms can also be obtained in a similar manner.

Moreover, since the nucleotide sequence of *the fasR* gene may differ depending on species or strains of coryneform bacteria, *the fasR* gene used for the present invention is not limited to those having the nucleotide sequence of the nucleotide numbers 506 to 967 of SEQ ID NO: 3 or that of SEQ ID NO: 5, and may be any of mutants and artificially modified ones encoding a protein having the amino acid sequence of SEQ ID NO: 4 or 6 including substitution, deletion, insertion or addition of one or several amino acid residues at one or more positions, so long as the function of the encoded FasR protein is maintained, namely, it can improve the sensitivity to surfactant by amplification thereof. Although the number of the "several" amino acid residues referred to herein may differ depending on positions in the three-dimensional structure or types of amino acid residues of the protein, it may be preferably 2 to 20, more preferably 2 to 10, particularly preferably 2 to 5. Moreover, such substitution, deletion, insertion, addition and inversion of amino acid residues as mentioned above include those caused by a naturally occurring mutation or variation based on difference of individuals or species of the microorganism containing the *fasR* gene.

The aforementioned substitution is preferably a conservative substitution which is a neutral mutation not causing functional change. Conservative substitutions include:
substitutions of Phe, Trp, and Tyr for each other in the case of aromatic amino acids, substitutions of Leu, Ile, and Val for each other in the case of hydrophobic amino acids, substitutions of Gln and Asn for each other in the case of polar amino acids, substitutions of Lys, Arg, and His for each other in the case of basic amino acids, substitutions of Asp and Glu for each other in the case of acidic amino acids, and substitutions of Ser and Thr for each other in the case of hydroxyl group-containing amino acids. More specifically, examples of the conservative mutations include: substitution of Ser or Thr for Ala, substitution of Gln, His, or Lys for Arg, substitution of Glu, Gln, Lys, His, or Asp for Asn, substitution of Asn, Glu, or Gln for Asp, substitution of Ser or Ala for Cys, substitution of Asn, Glu, Lys, His, Asp, or Arg for Gln, substitution of Gly, Asn, Gln, Lys, or Asp for Glu, substitution of Pro for Gly, substitution of Asn, Lys, Gln, Arg, or Tyr for His, substitution of Leu, Met, Val, or Phe for Ile, substitution of Ile, Met, Val, or Phe for Leu, substitution of Asn, Glu, Gln, His, or Arg for Lys, substitution of Ile, Leu, Val, or Phe for Met, substitution of Trp, Tyr, Met, Ile, or Leu for Phe, substitution of Thr or Ala for Ser, substitution of Ser or Ala for Thr, substitution of Phe or Tyr for Trp, substitution of His, Phe, or Trp for Tyr and substitution of Met, Ile, or Leu for Val.

Furthermore, as the *fasR* gene, also employable is a gene encoding a protein having a homology of 80% or more, preferably 90% or more, more preferably 95% or more, particularly preferably 97% or more, to the entire amino acid sequence of SEQ ID NO: 4 or 6 and capable of improving sensitivity to a surfactant of coryneform bacteria by enhancement of expression thereof. Moreover, since the degeneracy of gene changes depending on host to which the gene is introduced, the *fasR* gene may be one in which codons are replaced with other codons more easily used in the host into which *jasR* is introduced. The *fasR* gene may also be extended or deleted so as to extend or delete the protein on the N-terminus side or the C-terminus side, so long as it has the function of improving sensitivity of coryneform bacteria to a surfactant by amplification thereof. The length of the extension or deletion is, for example, 50 or less, preferably 20 or less, more preferably 10 or less, particularly preferably 5 or less, in terms of the number of amino acid residues. More specifically, it may be a protein having the amino acid sequence of SEQ ID NO: 4 of which 50 to 5 amino acid residues on the N-terminus side or 50 to 5 amino acid residues on the C-terminus side is deleted, or the amino acid sequence of SEQ ID NO: 4 which is extended by 50 to 5 amino acid residues on the N-terminus side or C-terminus side.

Such a gene homologous to the *fasR* gene can be obtained by, for example, modifying the nucleotide sequence of the nucleotide numbers 506 to 976 of SEQ ID NO: 3 or the nucleotide sequence of SEQ ID NO: 5 by site-specific mutagenesis so that amino acid residues of a specific site of the encoded protein includes substitution, deletion, insertion or addition of amino acid residues. Furthermore, it can also be obtained by the conventionally known mutagenizing treatments described below. The mutagenizing treatments include treating the nucleotide sequence of the nucleotide numbers 506 to 976 of SEQ ID NO: 3 or the nucleotide sequence of SEQ ID NO: 5 with hydroxylamine or the like in vitro, treating a microorganism such as a coryneform bacterium containing the gene with ultraviolet radiation or a mutagenizing agent used for usual mutagenizing treatment such as N-methyl-N'-nitro-N-nitrosoguanidine (NTG) or ethyl methanesulfonate (EMS). A mutation can be artificially introduced into *fasR* by gene recombination to obtain a highly active fasR by error-prone PCR, DNA shuffling or StEP-PCR (Firth AE, Patrick WM, Bioinformatics., 2005 Jun. 2, Statistics of protein library construction). Whether such a *fasR* homologous gene encodes a protein that improves sensitivity to a surfactant when expression amount thereof is increased can be confirmed by, for example, introducing the gene into a wild type strain of a coryneform bacterium, and examining whether the L-glutamic acid producing ability is improved in a medium to which a surfactant is not added.

The *fasR* gene also includes a DNA which is able to hybridize with the nucleotide sequence of the nucleotide numbers 506 to 976 of SEQ ID NO: 3, the nucleotide sequences of SEQ ID NO: 5, or a prove which can be prepared from the foregoing sequences under stringent conditions and encoding a protein which improve sensitivity to a surfactant of coryneform bacteria when expression thereof is enhanced. Here, the "stringent conditions" are conditions under which a so-called specific hybrid is formed, and a non-specific hybrid is not formed. Examples of the stringent conditions include those under which highly homologous DNAs hybridize to each other, for example, DNAs not less than 80, 90, 95, or 97% homologous, hybridize to each other, and DNAs less homologous than the above do not hybridize to each other, or conditions of washing once or preferably 2 or 3 times at a salt concentration and temperature corresponding to washing conditions in typical Southern hybridization, i.e., I x SSC, 0.1 % SDS at 60°C, preferably 0.1 x SSC, 0.1 % SDS at 60°C, more preferably 0.1 x SSC, 0.1% SDS at 68°C.

As the probe, a probe having a partial sequence of the nucleotide sequence of the nucleotide numbers 506 to 976 of SEQ ID NO: 3 or the nucleotide sequences of SEQ ID NO: 5 can also be used. Such a probe can be produced by PCR using oligonucleotides prepared on the basis of the nucleotide sequence of the nucleotide numbers 506 to 976 of SEQ ID NO: 3 or the nucleotide sequences of SEQ ID NO: 5 as primers and a DNA fragment containing the nucleotide sequence of the nucleotide numbers 506 to 976 of SEQ ID NO: 3 or the nucleotide sequences of SEQ ID NO: 5 as a template. When a DNA fragment having a length of about 300 bp is used as the probe, the washing conditions of hybridization are, for example, 2 x SSC, 0.1 % SDS at 50°C.

Enhancement of the expression of *the fasR* gene can be attained by increasing copy number *of the fasR* gene. For example, a recombinant DNA can be prepared by ligating a gene fragment containing *the fasR* gene with a vector functioning in coryneform bacteria, preferably such a multi-copy type vector, and introduced into a host having the aforementioned L-glutamic acid producing ability to transform it. Alternatively, the aforementioned recombinant DNA can be introduced into a wild-type coryneform bacterium to obtain a transformant, and then the L-glutamic acid producing ability can be imparted to the transformant. The increase of copy number can also attained by transferring one or more copies of a gene encoding *fasR* to a chromosome. Transfer of *the fasR* gene to a chromosome can be confirmed by performing Southern hybridization using a part of the *fasR* gene as a probe.

Enhancement of the expression of *the fasR* gene can also be attained by modifying an expression control sequence *of the fasR* gene. For example, it can be attained by replacing a promoter sequence *of the fasR* gene with a stronger promoter, or approximating a promoter sequence to a consensus sequence (International Patent Publication WO00/18935).

Methods for constructing a coryneform bacterium modified so that expression amount of *the fasR* gene increases are shown below. These methods can be performed by referring to manuals such as Molecular Cloning (Cold Spring Harbor Laboratory Press, Cold spring Harbor (USA), 2001).

Enhancement of the expression of *the fasR* gene can be attained by increasing copy number of the gene, and the increase of the copy number can be attained by amplifying the *fasR* gene using a plasmid as described below. First, *the fasR* gene is cloned from a chromosome of coryneform bacterium. Chromosomal DNA can be prepared from the bacterium as a DNA donor by, for example, the method of Saito and Miura (refer to H. Saito and K. Miura, Biochem. Biophys. Acta, 72, 619 (1963); Text for Bioengineering Experiments, Edited by the Society for Bioscience and Bioengineering, Japan, pp.97-98, Baifukan, 1992), or the like. Oligonucleotides used for PCR can be synthesized on the basis of known information such as mentioned above, and the *fasR* gene can be amplified by using, for example, the synthetic oligonucleotides of SEQ ID NOS: 1 and 2.

If a gene fragment containing the *fasR* gene amplified by PCR is ligated to a vector DNA autonomously replicable in a cell of *Escherichia coli* and/or coryneform bacteria to form a recombinant DNA, and this recombinant DNA is introduced into an *Escherichia coli* cell, the subsequent procedure becomes easy. Examples of the vector autonomously replicable in *Escherichia coli* cells include pUC19, pUC18, pHSG299, pHSG399, pHSG398, RSF1010, pBR322, pACYC184, pMW219 and so forth.

The aforementioned DNA is introduced into a vector which functions in coryneform bacteria. The vector which functions in coryneform bacteria is, for example, a plasmid that can autonomously replicate in coryneform bacteria. Specific examples of the plasmid that can autonomously replicate in coryneform bacteria include, for example, the plasmid pCRY30 described in Japanese Patent Laid-open No. 3-210184; plasmids pCRY21, pCRY2KE, pCRY2KX, pCRY31, pCRY3KE and pCRY3KX described in Japanese Patent Laid-open No. 2-72876 and U.S. Patent No. 5,185,262; the plasmids pCRY2 and pCRY3 described in Japanese Patent Laid-open No. 1-191686; pAM330 described in Japanese Patent Laid-open No. 58-67679; pHM1519 described in Japanese Patent Laid-open No. 58-77895; pAJ655, pAJ611 and pAJ 1844 described in Japanese Patent Laid-open No. 58-192900; pCG1 described in Japanese Patent Laid-open No. 57-134500; pCG2 described in Japanese Patent Laid-open No. 58-35597; pCG4 and pCG11 described in Japanese Patent Laid-open No. 57-183799; and pVK7 described in Japanese Patent Laid-open No. 10-215883.

Moreover, if a DNA fragment having an ability to make a plasmid autonomously replicable in coryneform bacteria is taken out from these vectors and inserted into the aforementioned vectors for *Escherichia coli,* they can be used as a so-called shuttle vector autonomously replicable in both of *Escherichia coli* and coryneform bacteria.

These vectors can be obtained from the deposited bacteria as follows. That is, cells collected in their exponential growth phase are lysed by using lysozyme and SDS, and centrifuged at 30000 x g. The supernatant obtained from the lysate is added with polyethylene glycol, fractionated and purified by cesium chloride-ethidium bromide equilibrium density gradient centrifugation.

In order to ligate the *fasR* gene and a vector that functions in coryneform bacteria to prepare a recombinant DNA, the vector is digested with a restriction enzyme providing an end matching the end of *the fasR* gene. This restriction site may be introduced into the synthetic oligonucleotides used for the amplification *of the fasR* gene beforehand. The ligation is usually performed by using a ligase such as T4 DNA ligase.

To introduce the recombinant DNA prepared as described above into a coryneform bacterium, any known transformation methods that have hitherto been reported can be employed. For instance, employable are a method of treating recipient cells with calcium chloride so as to increase permeability thereof for DNA, which has been reported for *Escherichia coli* K-12 (Mandel, M. and Higa, A., J. Mol. Biol., 53, 159 (1970)), and a method of preparing competent cells from cells which are at the growth phase followed by introducing the DNA thereinto, which has been reported for *Bacillus subtilis* (Duncan, C.H., Wilson, G.A. and Young, F.E., Gene, 1, 153 (1977)). In addition to these, also employable is a method of making DNA-recipient cells into protoplasts or spheroplasts, which can easily take up recombinant DNA, followed by introducing the recombinant DNA into the cells, which is known to be applicable to *Bacillus subtilis,* actinomycetes and yeasts (Chang, S. and Choen, S.N., Mol. Gen. Genet., 168, 111 (1979); Bibb, M.J., Ward, J.M. and Hopwood, O.A., Nature, 274, 398 (1978); Hinnen, A., Hicks, J.B. and Fink, G.R., Proc. Natl. Sci. USA, 75, 1929 (1978)). In addition, transformation of coryneform bacteria can also be performed by the electric pulse method (Japanese Patent Laid-open No. 2-207791) or conjugative transfer (Biotechnology (N Y), 1991 Jan, 9(1):84-7).

Increase of copy number of *the fasR* gene can also be achieved by introducing multiple copies of *the fasR* gene into a chromosomal DNA of coryneform bacteria. In order to introduce multiple copies of *the fasR* gene into a chromosomal DNA of coryneform bacteria, homologous recombination is carried out by using a sequence whose multiple copies present in the chromosomal DNA as targets. As sequences whose multiple copies present in the chromosomal DNA, repetitive DNA, and inverted repeats existing at the end of a transposable element can be used. Alternatively, as disclosed in Japanese Patent Laid-open No. 2-109985, it is also possible to incorporate *the fasR* gene into a transposon, and allow it to transfer to introduce multiple copies of the genes into the chromosomal DNA (Japanese Patent Laid-open Nos. 2-109985 and 7-107976; Mol. Gen. Genet., 245, 397-405 (1994); Plasmid, 2000 Nov, 44(3):285-91).

Moreover, it is also contemplated to introduce *the fasR* gene into a plasmid which contains a replication origin which cannot replicate in a host, or a plasmid which contains a replication origin which cannot replicate in a host and is capable of conjugative transfer to the host, thereby amplifying the gene on a chromosome. Examples of usable vectors include, for example, pSUP301 (Simo et al., Bio/Technology, 1, 784-791 (1983)), pK18mob or pK19mob (Schaefer et al., Gene, 145, 69-73 (1994)), pGEM-T (Promega, Madison, WI, USA), pCR2.1-TOPO (Shuman, Journal of Biological Chemistry, 269: 32678-84 (1994); US-A5,487,993), pCR® Blunt (Invitrogen, Groningen, Netherlands; Bernard et al., Journal of Molecular Biology, 234:534-541 (1993)), pEM1 (Schrumpf et al., Journal of Bacteriology, 173: 1991, 4510-4516), pBGS8 (Spratt et al, 1986, Gene, 41:337-342), and so forth. A plasmid vector containing the *fasR* gene is transferred into a coryneform bacterium by conjugation or transformation. Methods for the conjugation are described in, for example, Schaefer et al. (Applied and Environmental Microbiology, 60, 756-759 (1994)). Methods for the transformation are described in, for example, Theirbach et al., Applied Microbiology and Biotechnology, 29, 356-362 (1988); Dunican and Shivinan, Bio/Technology 7, 1067-1070, (1989); and Tauch et al., FEMS Microbiological Letters, 123, 343-347 (1994).

Moreover, increase of the activity of the fasR can also be achieved by replacing an expression control sequence such as a promoter on a chromosomal DNA or a plasmid with a stronger promoter, modifying a factor which participates in expression control of *fasR,* for example, an operator or a repressor, or by ligating a potent terminator (Hamilton et al, Journal of Bacterology, 171:4617-4622). For example, lac promoter, *trp* promoter, *trc* promoter, PS2 promoter and so forth are known as strong promoters. Methods for evaluating potency of promoters and examples of potent promoter are described in the paper of Goldstein et al. (Prokaryotic promoters in biotechnology, Biotechnol. Annu. Rev., 1995, 1, 105-128), and so forth. Moreover, as disclosed in International Patent Publication WO00/18935, it is also possible to introduce nucleotide substitution for several nucleotides into a promoter region of an objective gene so as to approximate the promoter sequence to a consensus sequence and thereby modify it into a stronger one. For example, it is contemplated to substitute TTGACA or TTGCCA sequence for the -35 region, or TATAAT or TATAAC sequence for the -10 region. Furthermore, it is known that replacement of several nucleotides in a spacer between a ribosome binding site (RBS) and a start codon, especially in a region immediately upstream from the start codon, significantly affects the translation efficiency of mRNA, and it is also possible to modify these.

Furthermore, increase of the expression amount can also be achieved by extending lifetime of m-RNA, or by preventing decomposition of enzyme proteins in cells. Expression control regions of the *fasR* gene such as promoter in an upstream region can also be determined by using a promoter searching vector, gene analysis software such as GENETYX, or the like. By such promoter substitution or modification, expression of *the fasR* gene is enhanced. Substitution of an expression control sequence can be performed by using, for example, a temperature sensitive plasmid. Examples of the temperature sensitive plasmid for coryneform bacteria include p48K and pSFKT2 (Japanese Patent Laid-open No. 2000-262288), pHSC4 (French Patent Laid-open No. 2667875, 1992 and Japanese Patent Laid-open No. 5-7491) and so forth. These plasmids can autonomously replicate at least at 25°C, but cannot autonomously replicate at 37°C in coryneform bacteria. The modification of expression control sequence may be performed in combination with the increase of copy number *of the fasR* gene.

### <2> Production of L-glutamic acid

L-glutamic acid can be efficiently produced by culturing a coryneform bacterium obtained as described above in a medium to produce and accumulate L-glutamic acid in the medium and collecting L-glutamic acid from the medium.

As the medium used for the culture, an ordinary medium that contains a carbon source, a nitrogen source, an inorganic salt, and optionally organic micronutrients such as amino acids and vitamins can be used. Either a synthetic medium or a natural medium may be used. Any kinds of carbon and nitrogen sources may be used so long as they can be utilized by the strain being cultured.

Saccharides such as glucose, glycerol, fructose, sucrose, maltose, mannose, galactose, starch hydrolysate, molasses, and so forth may be used as the carbon source. In addition, organic acids such as acetic acid and citric acid, and alcohols such as ethanol may also be used alone or in combination with other carbon sources. Ammonia, ammonium salts such as ammonium sulfate, ammonium carbonate, ammonium chloride, ammonium phosphate, and ammonium acetate, nitrates, and so forth may be used as the nitrogen source. Amino acids, vitamins, fatty acids, nucleic acids, and peptone, casamino acid, yeast extract, and soybean protein decomposition products which contain these substances may be used as the organic micronutrients. When an auxotrophic mutant strain that requires an amino acid etc. for growth is used, such a required nutrient is preferably added. Phosphates, magnesium salts, calcium salts, iron salts, manganese salts, and so forth can be used as inorganic salts.

In the case of the present invention, since the amount of surfactant required for induction of L-glutamic acid production can be reduced by amplifying the *fasR* gene, the medium may contain biotin at a high concentration. The medium desirably contains biotin at a concentration is 50 µg/L or higher, preferably 100 µg/L or higher, more preferably 200 µg/L or higher.

Aerobic culturing is performed by controlling the fermentation temperature to 20 to 45°C and adjusting the pH of the culture medium to 3 to 9. When the pH decreases during the culture, the medium is neutralized by adding alkali such as calcium carbonate or ammonia gas. Culture for about 10 to about 120 hours results in accumulation of a marked amount of L-glutamic acid in the medium.

Furthermore, the culture can also be performed with precipitating L-glutamic acid in the medium by using a liquid medium adjusted to be under such a condition that L-glutamic acid is precipitated in the medium. Examples of the condition under which L-glutamic acid precipitates include pH 5.0 to 4.0, preferably pH 4.5 to 4.0, more preferably pH 4.3 to 4.0, particularly preferably pH 4.0.

As for the method of collecting L-glutamic acid from the medium after the culture, collection may be performed by a known collection method. For example, the collection is attained by a method of removing cells from the medium, and causing crystallization by concentration, ion exchange chromatography, or the like. When the culture is performed under a condition under which L-glutamic acid precipitates, L-glutamic acid precipitated in the medium can be collected by centrifugation or filtration. In this case, L-glutamic acid dissolving in the medium may be precipitated and then separated together.

### Examples

Hereafter, the present invention will be specifically explained with reference to the following examples. However, the present invention is not limited to the following examples.

### Example 1: Preparation of fasR-amplified strain of C. glutamicum ATCC 13869

A plasmid for amplifying *the fasR* gene was prepared. Since the total genome sequence of *C*. *glutamicum* had been already determined, a chromosome was extracted from *C. glutamicum* ATCC13869 by using Bacterial Genomic DNA Purif. Kit (MS Techno Systems), and PCR was performed using this chromosome as a template and a combination of the primers of SEQ ID NOS: 1 and 2 to amplify a fragment of about 900 bp. PCR was performed by using Pyrobest polymerase (Takara Bio Inc.) with a cycle of denaturation at 98°C for 10 seconds, annealing at 55°C for 30 seconds and extension at 72°C for 2 minutes, which was repeated 25 times. The primers of SEQ ID NOS: 1 and 2 were added with the KpnI sequence at the 5'-end and designed to amplify the region of the nucleotide numbers 111 to 1010 of SEQ ID NO: 3.

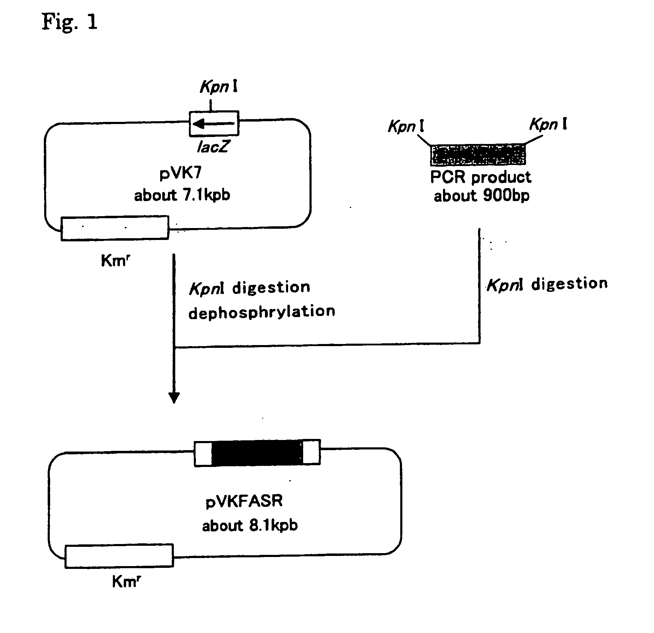

The amplified fragment was completely digested with *Kpn*I, and ligated to the pVK7 vector (described in Japanese Patent Laid-open No. 10-215883) similarly completely digested with *Kpn*I (Ligation Kit Ver. 2 (Takara Bio Inc.) was used) to construct pVKFASR (the construction scheme is shown in Fig. 1).

pVKFASR was introduced into C. *glutamicum* ATCC13869 by the electric pulse method (Japanese Patent Laid-open No. 2-207791), and the cells were applied to the CM-2B agar medium (10 g/l of polypeptone, 5 g/l of yeast extract, 5 g/l of NaCl, 10 µg/l of biotin, 20 g/l of agar, adjusted to pH 7.0 with KOH) containing 25 µg/ml of kanamycin. The strain that appeared was designated ATCC13869/pVKFASR as a fasR-amplified strain.

### Example 2: Confirmation of glutamic acid producing ability of ATCC 13869/pVKFASR strain

Glutamic acid production ability of the ATCC13869/pVKFASR strain was examined by performing culture using a Sakaguchi flask. A control strain, ATCC13869/pVK7, which was obtained by introducing the vector into the wild strain, and the ATCC13869/pVKFASR strain as the *fasR*-amplified strain were cultured at 31.5°C in the CM-2B agar medium (10 g/l of polypeptone, 5 g/l of yeast extract, 5 g/l of NaCl, 10 µg/l of biotin, 20 g/l of agar, adjusted to pH 7.0 with KOH) containing 25 µg/ml of kanamycin for one whole day, and inoculated into 20 ml of a seed culture medium (80 g/l of glucose, 30 g/l of ammonium sulfate, 1 g/l of KH₂PO₄, 0.4 g/l of MgSO₄· 7H₂O, 0.01 g/l of FeSO₄- 7H₂O, 0.01 g/l of MnSO₄· 4-5H₂O, 200 µg/l of vitamin B1, 0.48 g/l of soy bean protein hydrolysate, 300 µg/l of biotin, adjusted to pH 8.0 with KOH). One g of calcium carbonate which was subjected to dry heat sterilization beforehand was added to the medium, and then culture was performed at 31.5°C with shaking at a velocity of 115 rpm. After it was confirmed that the total sugar was completely consumed, 1 ml of the seed culture medium was inoculated into 20 ml of a main culture medium (80 g/l of glucose, 30 g/l of ammonium sulfate, 1 g/l of KH₂PO₄, 0.4 g/l of MgSO₄-7H₂O, 0.01 g/l of FeSO₄· 7H₂O, 0.01 g/l of MnSO₄·4-5H₂O,200 µg/l of vitamin B1, 0.48 g/l of soy bean protein hydrolysate, 300 µg/l of biotin, adjusted to pH 8.0 with KOH). One g of calcium carbonate which was subjected to dry heat sterilization beforehand was added to the medium, and then culture was performed at 31.5°C with shaking at a velocity of 115 rpm. After 2.5 hours from the start of the culture, 0.01, 0.1 or 1 g/l of Tween 40 (Sigma) was added, or Tween 40 was not added. The cell amounts (absorption was measured at 620 nm), the accumulated glutamic acid amounts, and the remained saccharide amounts after 41 hours are shown in Table 1.

As a result, the ATCC13869/pVKFASR strain accumulated about 10 g/l of glutamic acid under the conditions that the surfactant was not added or added in a small amount, under which the ATCC13869/pVK7 strain did not accumulate glutamic acid. From the above results, it was confirmed that amplification of the *fasR* gene was effective for completely eliminating need of surfactant or reducing the amount of surfactant, which is usually necessary for induction of glutamic acid production.

**Table 1: Glutamic acid production amounts of fasR-amplified strain**

| Concentration of surfactant | Strain | OD620nm | Glutamic acid (g/l) | Remaining saccharide (g/l) |
|---|---|---|---|---|
| 0 g/l | ATCC13869/pVK7 | 80.68 | 0.0 | 0.0 |
| | ATCC13869/pVKFASR | 64.41 | 11.0 | 0.0 |
| 0.01 g/l | ATCC13869/pVK7 | 79.15 | 0.0 | 0.0 |
| | ATCC13869/pVKFASR | 66.05 | 6.8 | 0.0 |
| 0.1 g/l | ATCC13869/pVK7 | 78.49 | 0.0 | 0.0 |
| | ATCC13869/pVKFASR | 60.59 | 11.8 | 0.0 |

### Example 3: Change of sensitivity of ATCC13869/pVKFASR strain to surfactant

Since glutamic acid production was induced in the *fasR*-amplified strain only by adding the surfactant at a low concentration, it was estimated that the sensitivity of the strain to a surfactant increased as compared with the control strain (vector-introduced strain ATCC13869/pVK7). Therefore, the sensitivity of the *fasR*-amplified strain to a surfactant was examined. Cells grown at 31.5°C on the CM-2B agar medium over one whole day were inoculated to the CM-2B liquid medium, and Tween 40 was added to the medium at a concentration of 0.01, 0.1, 0.25 or 0.5 g/l, or was not added. By performing test tube culture, absorption at 620 nm (OD 620 nm) was measured over time. Specific growth rate was calculated, and the results are shown in Fig: 2. Marked decrease in the specific growth rate was observed for the *fasR*-amplified strain with addition of 0.01 g/L of the surfactant, and thus it was confirmed that the fasR gene was a gene which changed the sensitivity to a surfactant.

## Claims

1. A method for producing L-glutamic acid, comprising:
(A) culturing a coryneform bacterium in a medium, and
(B) collecting L-glutamic acid from the medium, wherein said coryneform bacterium is modified so that the expression of the fasR gene is enhanced by increasing the copy number of the gene or modifying an expression control sequence of the gene as compared to a parent strain, such that said bacterium is capable of producing L-glutamic acid in a medium containing biotin at a concentration of 50 µg/l or higher and not containing a biotin activity suppressing agent selected from the group consisting of surfactant and penicillin, and wherein the fasR gene is selected from the group consisting of:
(a) a DNA comprising nucleotides 506 to 976 in SEQ ID NO: 3 or nucleotides 1 to 474 in SEQ ID NO: 5,
(b) a DNA which is able to hybridize with nucleotides 506 to 976 in SEQ ID NO: 3 or nucleotides 1 to 474 in SEQ ID NO: 5 under stringent conditions comprising washing at 0.1 x SSC, 0.1% SDS at 60°C.

2. The method according to claim 1, wherein said bacterium is Corynebacterium glutamicum.

3. *The method according to any one of items 1 to 2, wherein said fasR genre has a homology of 95% or more to the entire amino acid sequence of SEO ID NO: 4.*

## Patentansprüche

1. Verfahren zur Herstellung von L-Glutaminsäure, umfassend:
(A) Kultivierung eines coryneformen Bakteriums in einem Medium, und
(B) Gewinnung der L-Glutaminsäure aus dem Medium, wobei das genannte coryneforme Bakterium modifiziert ist, so dass die Expression des fasR-Gens gesteigert ist durch die Erhöhung der Kopienzahl des Gens oder die Modifizierung einer Expressions-Kontrollsequenz des Gens im Vergleich zum Vorläuferstamm, so dass das Bakterium in der Lage ist, L-Glutaminsäure in einem Medium herzustellen, das Biotin in einer Konzentration von 50 µg/l oder höher enthält und kein Biotinaktivitäts-supprimierendes Agens enthält, das ausgewählt ist aus der Gruppe bestehend aus Tensid und Penicillin, und wobei das fasR-Gen ausgewählt ist aus der Gruppe bestehend aus:
(a) einer DNA, die die Nukleotide 506 bis 976 in SEQ ID NO: 3 oder die Nukleotide 1 bis 474 in SEQ ID NO: 5 umfasst,
(b) einer DNA, die in der Lage ist, mit den Nukleotiden 506 bis 976 in SEQ ID NO: 3 oder den Nukleotiden 1 bis 474 in SEQ ID NO: 5 unter stringenten Bedingungen zu hybridisieren, die Waschen bei 0,1 x SSC, 0,1 % SDS bei 60°C umfassen.

2. Verfahren gemäß Anspruch 1, wobei das Bakterium Corynebacterium glutamicum ist.

3. Verfahren gemäß einem der Ansprüche 1 bis 2, wobei das fasR-Gen eine Homologie von 95 % oder mehr mit der gesamten Aminosäuresequenz von SEQ ID NO: 4 hat.

## Revendications

1. Procédé pour produire de l'acide L-glutamique comprenant :
(A) la culture d'une bactérie coryneforme dans un milieu, et
(B) la collecte de l'acide L-glutamique à partir du milieu, où ladite bactérie coryneforme est modifiée de sorte que l'expression du gène fasR est amplifiée par augmentation du nombre de copies du gène ou modification d'une séquence de contrôle de l'expression du gène par rapport à une souche mère, de sorte que ladite bactérie est capable de produire de l'acide L-glutamique dans un milieu contenant de la biotine à une concentration de 50 µg/l ou plus et ne contenant pas d'agent supprimant l'activité de la biotine choisi dans le groupe consistant en un tensioactif et la pénicilline, et où le gène fasR est choisi dans le groupe consistant en :
(a) un ADN comprenant les nucléotides 506 à 976 dans SEQ ID NO : 3 ou les nucléotides 1 à 474 dans SEQ ID NO : 5,
(b) un ADN qui est capable de s'hybrider avec les nucléotides 506 à 976 dans SEQ ID NO : 3 ou les nucléotides 1 à 474 dans SEQ ID NO : 5 dans des conditions stringentes comprenant un lavage à 0,1 x SSC, SDS 0,1 % à 60°C.

2. Procédé selon la revendication 1 où ladite bactérie est Corynebacterium glutamicum.

3. Procédé selon l'une quelconque des revendications 1 à 2 où ledit gène fasR a une homologie de 95 % ou plus avec la séquence d'aminoacides entière de SEQ ID NO : 4.
